# EUROPEAN PATENT SPECIFICATION

(11) **EP 0 821 657 B1**
(45) Date of publication and mention of the grant of the patent: **22.09.1999**
(21) Application number: 96911147.5
(22) Date of filing: 12.04.1996
(51) Int. Cl.: C02F 1/32, A61L 2/10, A61L 9/20

(54) **UV-REFLECTOR FORMING A PART OF A UV-DEVICE FOR PURIFICATION OF A LIQUID OR A GAS, PREFERABLY WATER**
UV-REFLEKTOR, INTEGRIERT IN EINE UV-ANLAGE ZUR GAS- ODER FLÜSSIGKEITSREINIGUNG, BEVORZUGT VON WASSER
REFLECTEUR A ULTRAVIOLETS INTEGRE A UN DISPOSITIF A ULTRAVIOLETS DE PURIFICATION DE GAZ OU DE LIQUIDE, ET DE PREFERENCE DE L'EAU

(30) Priority: 21.04.1995 SE 9501467
(43) Date of publication of application: 04.02.1998
(73) Proprietor: AKTIEBOLAGET ELECTROLUX, 105 45 Stockholm (SE)
(72) Inventor: HAEGERMARCK, Anders, S-142 63 Trangsund (SE)
(74) Representative: Erixon, Bo
(86) International application number: SE9600483
(87) International publication number: WO9633135

(56) References cited:
- WO-A-90/06899
- DE-A- 2 146 504
- FR-A- 2 555 567

## Description

The present invention relates to a UV-reflector forming a part of a UV-device for purification of a liquid, preferably water, the present UV-reflector having at least partly an elliptic cross-sectional shape, said UV-device also including a source of light located in the area of one focal axis of the ellipse, said source of light emitting UV-light, and a tube located in the other focal axis of the ellipse, said tube being transparent for UV-light, the liquid being purified when passing through said tube by radiation of light from the UV source of light. The invention also relates to a UV-device including the UV-reflector according to the present invention.

UV-reflectors/UV-devices of the type mentioned above are previously known from e.g. DE-OS-2 146 504 and WO 90/06899. Generally it can be said that by using the elliptic cross-sectional shape the emitted UV-light is highly concentrated to the tube, normally made out of quartz glass, that the liquid passes through. However, a part of the UV-light that is emitted in the area between the UV source of light and the closest part of the periphery of the ellipse will be reflected back to the UV source of light and thus not reflected in direction to the light transparent tube. The corresponding is also valid for certain light beams that are directed to that portion of the ellipse that is closest to the quartz tube, i.e. said light beams are not reflected in direction to the quartz tube but are bypassing the quartz tube. This drawback will be further dealt with in the description below dealing with the embodiments.

The aim of the present invention is to present a UV reflector being free from the drawbacks discussed above, i.e. all the UV-light emitted from the source of light is in principal reflected to the quartz tube. The invention is realized by a UV-reflector that has been given the characterizing features of the appending claims.

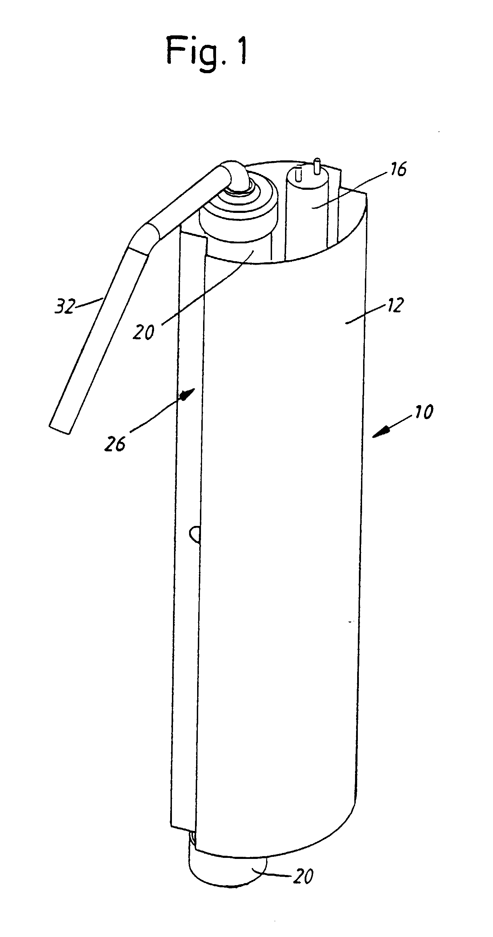

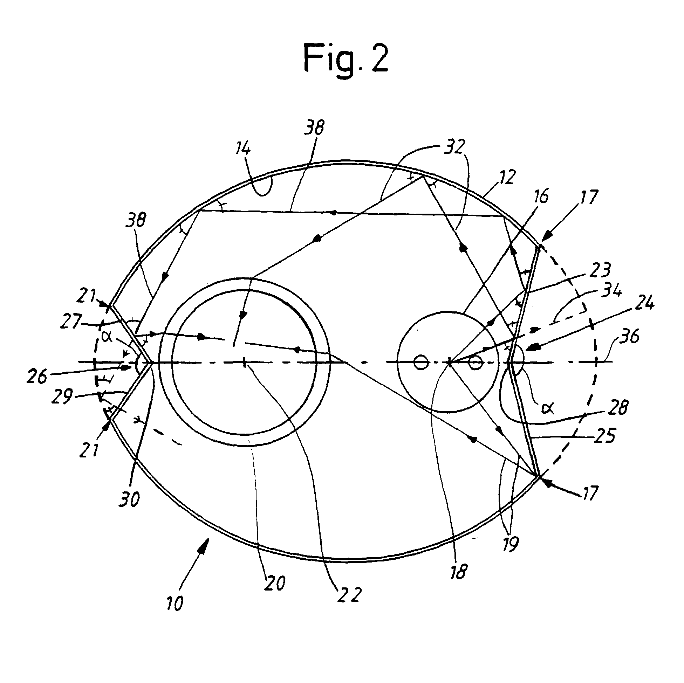

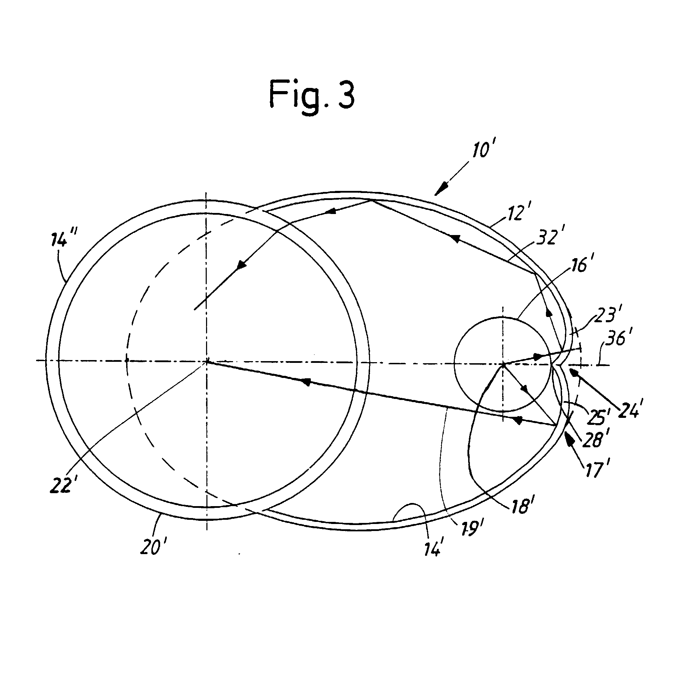

Below embodiments of the invention will be described, reference being made to the accompanying drawings, where Fig.1 shows a schematic perspective view of a UV-device according to the present invention, said device including a UV-reflector according to the present invention; Fig.2 shows a top view of the UV-device according to Fig.1, however for the sake of clarity certain details have been omitted; and Fig.3 shows a schematic top view of an alternative embodiment of a UV-device/UV-reflector according to the present invention.

The UV-device shown in Figs.1 and 2 is generally designated by 10 and includes a UV-reflector 12 that in cross-section has an elliptical basic shape and preferably is manufactured out of aluminium. In order to achieve maximum reflection of the UV-light the reflecting interior surface 14 of the UV-reflector 12 is preferably brightened through anodic oxidation. The UV-device 10 also includes a UV source of light 16 emitting said UV-light. The centre 18 of the the UV source of light 16 is located in one focal axis of the ellipse.

A tube 20 that is transparent for UV-light, said tube 20 normally being made out of quartz glass, is located with its centre 22 in the other focal axis of the ellipse. Said tube 20 will below in the description be called quartz tube. Within the scope of the invention it is possible that said tube e.g. is manufactured out of TEFLON®, however in such a case the tube should have very thin walls since TEFLON® to a larger extent absorbs the passing light compared to glass. Since such a tube is subjected only to an internal liquid pressure it can have very thin walls, i.e. a value of 0.2 mm seems suitable. A tube out of TEFLON® is preferable in the respect that sediments do not easily adhere to such a material and that it is not aged equally fast as glass. It should be noticed that when glass is aged its light transmission ability decreases.

The reflector 12 has a certain lengthwise extension and the UV source of light 16 and the quartz tube 20 extend in principal along the entire length of the reflector 12, i.e. the UV source of light 16 and the quartz tube 20 have about the same length.

From Figs.1 and 2 it is evident, seen in cross-section, that in the areas of its end portions the UV-reflector 12 is provided with a folding 24 and 26 respectively. Alternatively it can be said that the foldings 24, 26 are located adjacent to where the major axis 36 of the ellipse intersects the periphery of a conventional ellipse, see the broken periphery in Fig.2. The folding 24 includes two side surfaces 23 and 25, that meet in a point 28 while the folding 26 includes two side surfaces 27 and 29 that meet in a point 30. The point 28 and 30 respectively of the folding 24 and 26 respectively is located between the periphery of an imaginary (se the broken line in Fig.2), ordinary ellipse and the UV source of light 16 and the quartz tube 20 respectively, preferably as close to the UV source of light 16 as possible. In the disclosed embodiment the side surfaces 23, 25 are planar, however within the scope of the invention it is possible that the side surfaces are bending inwardly or outwardly. The side surfaces 23, 25 and 27, 29 respectively include an angle a that is in the interval 90°-180°. As is evident from Fig.2 the angle a can have different values for the foldings 24 and 26 respectively.

The length of the side surfaces 23, 25 and 27, 29, seen in cross-section of the reflector 12, depends on a number of different parameters, e.g. the shape of the ellipse and the diameter of the UV source of light 16. As regards the folding 24 its side surfaces 23 and 25 respectively join the periphery of the ellipse in an area 17 located directly beside a place where a beam 19, coming from the centre 18 of the UV source of light 16, is reflected against the elliptic part of the UV-reflector 12 and by-passes the UV source of light 16 without interfering said source of light 16. Said area of connection 17 for the folding/indentation 24 is thus located closer to the major axis 36 of the ellipse than the place where the beam 19 is reflected. It is realized that the location of the connection 17 for the folding/indentation 24 depends from several different factors, e.g. the diameter of the UV source of light 16 and the shape of the ellipse. The folding 26 is of less importance than the folding 24. The location of the connection 21 of the side surfaces 27, 29 to the elliptic part of the UV-reflector 12 is also defined by the angle a, the diameter of the tube 20 and how close to said tube 20 the point 30 of the folding 26 should be located. The definite design of the foldings 24 and 26 is normally defined empirically. Naturally the foldings 24 and 26 can not be given such small dimensions that they have no practical effect.

The UV-device 10 is intended to be incorporated in a device for purification, preferably sterilization, of a liquid or a gas, preferably water. Such a device includes apart from said UV-device also a filtering device that the liquid or gas passes through before it enters the UV-device 10. Said filtering device is not shown in the accompanying figures since the filtering device does not constitute a part of the present invention but filtering devices of different designs can be used together with the UV-reflector/UV-device according to the present invention. Normally such a filtering device includes a combined carbon/particle filter and possibly also anion exchanger and cation exchanger depending on the degree of hardness of the water. Further components can be included in the filtering device when special contaminations need to be removed from the liquid that is to be purified/sterilized.

When the liquid has passed through the filtering device it enters the quartz tube 20 at its lower end, the connection between the filtering device and the quartz tube 20 being designed in a suitable way. However, it should be arranged that the liquid is supplied eccentrically and tangentially to the quartz tube 20. Thereby it is guaranteed that turbulence occurs when the liquid enters the quartz tube 20, this supporting that all parts of the liquid being subjected to the same UV light radiation.

The liquid rising through the quartz tube 20 is subjected to radiation of UV-light that is emitted from the UV source of light 16. Normally this radiation of UV-light brings about a sterilization of the liquid passing through the quartz tube 20, said liquid being discharged via an outlet pipe 32, see Fig.1.

As mentioned above, the UV source of light 16 and the quartz tube 20 have their centra in the two focal axes of the ellipse. Thereby most of the UV-light emitted from one focal axis will be reflected to the other focal axis. Due to the design of the reflector 12 according to the present invention almost all of the light that is emitted from the source of light 16 will be reflected to the quartz tube 20, this being an substantial improvement compared to the prior art where the ellipse has a conventional design, see the broken lines in Fig.2. To explain this a full line light beam 32 is shown, said light beam being reflected against one side surface 23 of the folding 24 and then a further reflection against the interior surface 14 of the reflector 12 before the beam 32 enters into the quartz tube 20. When the light beam 32 passes through the wall of the quartz tube 20, said light beam 32 is refracted towards the centre of the quartz tube 20. If the folding 24 is not present the dotted prolongation 34 of the light beam 32 will be refleted against the conventional, dotted ellipse. It is at once realized that said dotted light beam 34 will be reflected back into the UV source of light 16. In a corresponding way a light beam 38 can be studied, said light beam by-passing the quartz tube 20 and is reflected against the side surface 27 of the folding 26 and then further into the quartz tube 20. If the folding 26 is not present the light beam 38 will be reflected against the broken line portion and then by-passing the quartz tube 20. Through the examples of light beams given above it is realized that the provision of the foldings 24 and 26 results in an improved efficiency as regards the ratio between light emitted from the UV source of light 16 and light supplied to the quartz tube 20.

Fig.3 shows an alternative embodiment of a UV-reflector 12' according to the present invention, the UV-reflector 12' in a common way forming a part of a UV-device 10'. The UV-reflector 12' has, in cross-section, an elliptical basic shape and a UV source of light 16' has its centre 18' located in one of the focal axes of the ellipse. The quartz tube 20' intersects the reflector 12' and the portion of the quartz tube 20' that is located outside the reflector 12' serves as reflector. To achieve this the exterior surface of the portion of the quartz tube 20' located outside the ellipse is provided with a reflecting layer 14" or a reflector in the shape of a portion of a cylinder, said reflector being in direct contact with the exterior glass surface of the quartz tube 20'. Also the rest of the reflector 12' is, correspondingly to the embodiment according to Figs. 1 and 2, provided with a reflecting interior surface 14'. This is preferably effected by brightening through anodic oxidation of the interior surface of the reflector 12' that is made out of aluminium. Correspondingly to the embodiment according to Figs.1 and 2 the centre 22' of the quartz tube 20' is located in the other focal axis of the ellipse that is completed through the broken periphery in Fig.3.

As is evident from Fig.3 the reflector 12' is provided with a folding/an indentation 24' only in the area where the major axis 36' intersects the periphery of a conventional ellipse, see the broken periphery. The point 28 of the folding/indentation 24' is located between said imaginary, broken periphery and the source of light 16', preferably as close to the source of light 16' as possible. In the embodiment according to Fig.3 the side surfaces 23' and 25' of the folding/indentation 24' are bending outwardly in relation to the interior of the reflector 12'. Empirically it has shown that such a bending of the side surfaces is preferable for the efficiency of the reflector 12'. As pointed out above in connection with Figs.1 and 3, within the scope of the invention the side surfaces 23' and 25' of the folding/indentation 24' can be either planar or bending.

The folding 24' of the embodiment according to Fig.3 has principally the same function as the folding 24 of the embodiment according to Figs.1 and 2. Therefore reference is made to the functional description made above concerning the benefits that is achieved from the folding according to the present invention.

In Fig.3 a light beam 32' has been introduced, said beam 32' being reflected against one side surface 23' of the folding 24' and then two further times against the interior surface 14' of the reflector 12' before the beam 32' enters into the quartz tube 20'. If this light beam 32' instead would have been reflected the first time against the interior surface of a conventional ellipse, this being indicated in Fig.3, a reflection back into the UV source of light 16' would have occured.

Correspondingly to the embodiment according to Fig.2 the folding/indentation 24' joins the periphery of the ellipse in the area 17' that is located directly adjacent a place where a light beam 19' emitting from the centre 18' of the UV source of light 16' is reflected against the interior surface 14' of the reflector 12' and by-passes the UV source of light 16' without interferring said source of light 16'. The area 17' is thus located closer to the major axis 36' than said reflection place for the light beam 19'.

The embodiment according to Fig.3 is extremely compact, i.e. it requires relatively limited space. This is due since the quartz tube 20' is partly integrated with the reflector 12'.

## Claims

1. UV-device (10;10') for purification of a liquid or a gas, preferably water, said UV-device (10;10') including a UVreflector (12;12') having at least partly an elliptical cross-sectional shape, said UV-device (10;10') including also a light source (16;16') located in the area of one focal axis of the ellipse, said source of light (16;16') emitting UV- light, and a tube (20;20') located in the area of the other focal axis of the ellipse, said tube (20;20') being transparent for UV light, that purification of the liquid or gas takes place during passage through said tube (20;20') by radiation of the liquid or gas using light from the UV source of light (16;16'),
**characterized in** that the reflector (12;12'), seen in cross-section, is provided with a folding/an indentation (24;24') in connection with the end portion of the reflector (12;12') being closest to the UV source of light (16;16').

2. UV-device according to claim 1,
**characterized in** that the folding/indentation (24;24') extends along almost the entire length of the UV-reflector (12;12'), and that side surfaces (23,25,27,29;23',25') of the folding/indentation join the elliptic part of the UV-reflector at a distance from the major axis (36;36') of the ellipse.

3. UV-device according to claim 1 or 2,
**characterized in** that the folding/indentation (24;24') has a point (28;28') that is directed towards the closest focal axis (18;18'), and that the folding/indentation (24;24') is symmetrical relative to the major axis (36;36') of the ellipse.

4. UV-device according to any of the preceding claims,
**characterized in** that the folding/indentation (24;24') joins the periphery of the ellipse in areas (17;17') located directly adjacent places of the periphery of the ellipse where a UV light beam (19;19') from the centre (18;18') of the UV source of light (16;16') is reflected and by-passes the UV source of light (16;16') without interferring said source of light (16;16'), said areas (17;17') being located closer to the major axis (36;36') of the ellipse than said places of reflection.

5. UV-device according to any of the preceding claims,
**characterized in** that, seen in cross-section, the reflector (12) has a folding/an indentation (26) in connection with the end portion located closest to the tube (20).

6. UV-device according to any of claims 1-4,
**characterized in** that, seen in cross-section, the side surfaces (23,25;23',25') of the folding /indentation (24;24') are planar or bending.

7. UV-device according to any of the preceding claims,
**characterized in** that the point (28;28') of the folding/indentation (24;24') is located directly adjacent to the UV source of light (16;16').

8. UV-device according to any of the preceding claims,
**characterized in** that, seen in cross-section, the reflector (12'), in the area of one end portion, includes a circular portion that is connected to an elliptical portion.

## Patentansprüche

1. UV-Anlage (10; 10') zur Reinigung einer Flüssigkeit oder eines Gases, vorzugsweise von Wasser, wobei die UV-Anlage (10; 10') einen UV-Reflektor (12; 12') aufweist, welcher zumindest teilweise eine elliptische Querschnittsform besitzt, und die UV-Anlage (10; 10') auch eine Lichtquelle (16; 16') aufweist, die in dem Bereich einer Brennachse der Ellipse angeordnet ist, wobei die Lichtquelle (16; 16') UV-Licht ausstrahlt, sowie ein Rohr (20, 20'), welches in dem Bereich der anderen Brennachse der Ellipse angeordnet ist, wobei das Rohr (20; 20') für UV-Licht transparent ist, und die Reinigung der Flüssigkeit oder des Gases während des Durchganges durch das Rohr (20; 20') durch Bestrahlung der Flüssigkeit oder des Gases unter Verwendung des Lichtes von der UV-Lichtquelle (16; 16') erfolgt,
**dadurch gekennzeichnet, daß** der Reflektor (12; 12'), im Querschnitt betrachtet, mit einer Einfaltung/einer Einkerbung (24; 24') in Verbindung mit dem Endbereich des Reflektors (12; 12'), welcher der UV-Lichtquelle (16; 16') am nächsten liegt, versehen ist.

2. UV-Anlage nach Anspruch 1, **dadurch gekennzeichnet, daß** die Einfaltung/Einkerbung (24; 24') sich nahezu über die gesamte Länge des UV-Reflektors (12; 12') erstreckt, und daß die Seitenflächen (23, 25, 27, 29; 23', 25') der Einfaltung/Einkerbung sich an den elliptischen Teil des UV-Reflektors in einem Abstand von der Hauptachse (36; 36') der Ellipse anschließen.

3. UV-Anlage nach den Ansprüchen 1 oder 2, **dadurch gekennzeichnet, daß** die Einfaltung/Einkerbung (24; 24') einen Punkt (28; 28') aufweist, welcher zur nächsten Brennachse (18; 18') gerichtet ist, und daß die Einfaltung/Einkerbung (24; 24') relativ zur Hauptachse (36; 36') der Ellipse symmetrisch angeordnet ist.

4. UV-Anlage nach einem oder mehreren der vorhergehenden Ansprüche, **dadurch gekennzeichnet, daß** die Einfaltung/Einkerbung (24; 24') an den Umfang der Ellipse in Bereichen (17; 17') anschließt, welche direkt neben den Stellen auf dem Umfang der Ellipse liegen, an welchen ein UV-Lichtstrahl (19; 19') vom Zentrum (18; 18') der UV-Lichtquelle (16; 16') reflektiert wird und an der UV-Lichtquelle (16; 16') vorbeigeht, ohne die Lichtquelle (16, 16') zu berühren, wobei die Bereiche (17; 17') näher an der Hauptachse (36; 36') der Ellipse gelegen sind als die reflektierenden Stellen.

5. UV-Anlage nach einem oder mehreren der vorhergehenden Ansprüche, **dadurch gekennzeichnet, daß** der Reflektor (12) im Querschnitt gesehen, eine Einfaltung/eine Einkerbung (26) in Verbindung mit dem Endbereich aufweist, welcher zum Rohr (20) am nächsten gelegen ist.

6. UV-Anlage nach einem oder mehreren der Ansprüche 1 bis 4, **dadurch gekennzeichnet, daß** die Seitenflächen (23, 25; 23', 25') der Einfaltung/Einkerbung (24; 24') im Querschnitt eben oder gewölbt sind.

7. UV-Anlage nach einem oder mehreren der vorhergehenden Ansprüche, **dadurch gekennzeichnet, daß** der Punkt (28; 28') der Einfaltung/Einkerbung (24; 24') direkt neben der UV-Lichtquelle (16; 16') gelegen ist.

8. UV-Anlage nach einem oder mehreren der vorhergehenden Ansprüche, **dadurch gekennzeichnet, daß** der Reflektor (12') im Bereich eines Endabschnittes einen kreisförmigen Bereich aufweist, welcher mit einem elliptischen Bereich verbunden ist.

## Revendications

1. Dispositif à ultraviolets (10 ; 10') destiné à la purification d'un liquide ou d'un gaz, de préférence de l'eau, ledit dispositif à ultraviolets (10 ; 10') comprenant un réflecteur d'ultraviolets (12 ; 12') ayant au moins en partie une forme elliptique en coupe transversale, ledit dispositif à ultraviolets (10 ; 10') comprenant également une source lumineuse (16 ; 16') placée dans la zone d'un axe focal de l'ellipse, ladite source lumineuse (16 ; 16') émettant une lumière ultraviolette, et un tube (20 ; 20') placé dans la zone de l'autre axe focal de l'ellipse, ledit tube (20 ; 20') étant transparent pour la lumière ultraviolette, cette purification du liquide ou du gaz se produit lors du passage dans ledit tube (20 ; 20') par rayonnement du liquide ou du gaz utilisant la lumière provenant de la source de lumière ultraviolette (16 ; 16'), caractérisé en ce que le réflecteur (12 ; 12'), vu en coupe transversale, est muni d'un pliage/d'une indentation (24 ; 24') en relation avec la partie d'extrémité du réflecteur (12 ; 12') qui est la plus proche de la source de lumière ultraviolette (16 ; 16').

2. Dispositif à ultraviolets selon la revendication 1, caractérisé en ce que le pliage/l'indentation (24 ; 24') s'étend presque sur toute la longueur du réflecteur d'ultraviolets (12 ; 12'), et en ce que des surfaces latérales (23, 25, 27, 29 ; 23', 25') du pliage/de l'indentation rejoignent la partie elliptique du réflecteur d'ultraviolets à une certaine distance de l'axe majeur (36 ; 36') de l'ellipse.

3. Dispositif à ultraviolets selon la revendication 1 ou 2, caractérisé en ce que le pliage/l'indentation (24 ; 24') présente un point (28 ; 28') qui est dirigé vers l'axe focal le plus proche (18 ; 18'), et en ce que le pliage/l'indentation (24 ; 24') est symétrique par rapport à l'axe majeur (36 ; 36') de l'ellipse.

4. Dispositif à ultraviolets selon l'une quelconque des revendications précédentes, caractérisé en ce que le pliage/l'indentation (24 ; 24') rejoint la périphérie de l'ellipse dans des zones (17 ; 17') situées directement à proximité d'emplacements de la périphérie de l'ellipse où un faisceau de lumière ultraviolette (19 ; 19') provenant du centre (18 ; 18') de la source de lumière ultraviolette (16 ; 16') est réfléchi et évite la source de lumière ultraviolette (16 ; 16') sans interférer avec ladite source de lumière (16 ; 16'), lesdites zones (17 ; 17') étant placées plus près de l'axe majeur (36 ; 36') de l'ellipse que lesdits emplacements de réflexion.

5. Dispositif à ultraviolets selon l'une quelconque des revendications précédentes, caractérisé en ce que, vu en coupe transversale, le réflecteur (12) présente un pliage/une indentation (26) en relation avec la partie d'extrémité placée le plus près du tube (20).

6. Dispositif à ultraviolets selon l'une quelconque des revendications 1 à 4, caractérisé en ce que, vues en coupe transversale, les surfaces latérales (23, 25 ; 23', 25') du pliage/de l'indentation (24 ; 24') sont planes ou courbes.

7. Dispositif à ultraviolets selon l'une quelconque des revendications précédentes, caractérisé en ce que le point (28 ; 28') du pliage/de l'indentation (24 ; 24') est placé directement à proximité de la source de lumière ultraviolette (16 ; 16').

8. Dispositif à ultraviolets selon l'une quelconque des revendications précédentes, caractérisé en ce que, vu en coupe transversale, le réflecteur (12'), dans la zone d'une partie d'extrémité, comprend une partie circulaire qui est reliée à une partie elliptique.
